# EUROPEAN PATENT SPECIFICATION

(11) **EP 2 722 025 B1**
(45) Date of publication and mention of the grant of the patent: **12.10.2016**
(21) Application number: 13189764.7
(22) Date of filing: 22.10.2013
(51) Int. Cl.: A61F 9/007, A61M 13/00

(54) **Syringe for injecting a surgical gas**
Spritze zum Einspritzen von chirurgischem Gas
Seringue destinée à injecter un gaz chirurgical

(30) Priority: 22.10.2012 DE 202012104055 U; 29.11.2012 DE 202012104643 U
(43) Date of publication of application: 23.04.2014
(73) Proprietor: FLUORON GMBH, 89077 Ulm (DE)
(72) Inventor: Häring, Tim, 89284 Pfaffenhofen a.d. Roth (DE); Geuder, Volker, 89077 Ulm (DE); Ripke, Christiane, 890773 Ulm (DE)
(74) Representative: Schiweck, Weinzierl & Koch

(56) References cited:
- EP-A1- 2 020 245
- EP-B1- 2 319 566
- WO-A2-2004/071268
- FR-A1- 2 850 564
- US-A- 6 073 759
- US-A1- 2010 087 776
- US-B1- 6 599 280
- MAY-CHING HONG ET AL: "Primary gas tamponade in the management of macular hole with retinal detachment in highly myopic eyes", JOURNAL OF THE CHINESE MEDICAL ASSOCIATION, ELSEVIER (SINGAPORE) PTE LTD, HONG KONG BRANCH, HK, vol. 74, no. 3, 13 October 2010 (2010-10-13), pages 121-124, XP028167366, ISSN: 1726-4901, DOI: 10.1016/J.JCMA.2011.01.026 [retrieved on 2011-01-19]

## Description

### Field of the invention

The invention relates to a syringe according to the pre-characterizing clause of Claim 1. Such a syringe is known from European patent specification EP 2 319 566 B1. The invention further relates to a system consisting of a bag and a syringe packed inside. In particular, the invention relates to a ready-to-use gas tamponade system for medical applications. The tamponade is used to fill a natural cavity resulting e.g. from the shrinkage of a vitreous humour due to ageing, or an artificial cavity resulting e.g. from a vitrectomy.

### Background of the invention

Increasing human life expectancy is giving rise to an increase in age-related diseases such as retinal changes or retinal detachments, glaucoma, cataract, and age-related macular degeneration and diabetic retinopathy. Treatment of these and other eye diseases usually requires a vitrectomy (removal of the vitreous humour). The resulting cavity has to be refilled to prevent the vitreous chamber from collapsing. This is done using "heavy gases" such as SF₆, C₂F₆ or C₃F₈. The term "heavy gases" is generally used to refer to gases which have a markedly higher density than normal atmospheric air.

In most cases the therapeutic effect is due not to the gas itself, but rather to the gas-liquid interface. This surface tension prevents gas from entering the subretinal space through a hole in the retina; in addition, the hole in the retina is expanded, preventing further liquid from entering the subretinal space.

After injection of the heavy gas, O₂ and CO₂ start to diffuse out of the blood and into the eye, increasing the volume of the gas bubble. A diffusion equilibrium is reached after a few hours for O₂ und CO₂, but only after a few days for N₂. The heavy gases are absorbed through the retina via the choroid membrane and, depending on the type, leave the eye within 1 to 2 or 4 weeks. In most cases to date, the gases used in surgery, such as SF₆, C₃F₈ or C₂F₆, have been transferred directly during the operation from a steel cylinder into the medium to be used. US patent 6,866,142 B2 or 6,073,759 A describes a system consisting of an already gas-filled disposable syringe which, for better gas tightness, is stored in a container filled with the same gas as the disposable syringe.

US patent 6,599,280 B1 and published specification FR 2 020 245 A both describe a surgical kit with a separate gas reservoir, a syringe and other components usable in combination with the syringe, which are housed in a pack.

International patent application WO 2004/071268 A2 discloses a gas accumulator for storing a predetermined gas quantity and for releasing said gas for medical use. The aim of the disclosed gas accumulator is to avoid the use of a non-controlled mixture of a gas with ambient air during the storage or extraction thereof by means of the inventive gas accumulator and to carry out a simple and reliable sterilisation of said gas. For this purpose, said gas accumulator is provided with an adsorbent for adsorbing a predetermined gas quantity to be extracted by desorption at an ambient temperature for a medical, in particular ophthalmological use, such as for an intraocular gas tamponade.

US patent application US 2010/0087776 discloses a system for filling a syringe with a small amount of a precisely mixed medical gas mixture of gases used in ophthalmic surgeries such as SF₆ and C₃F₈, the system comprising two or more sources for gas, a device for mixing the gases, a means for connecting the gas sources to the device for mixing the gases, a filter system and a syringe.

All the systems listed above have several disadvantages.
- In all the systems, SF₆, C₂F₆ and C₃F₈ are released into the environment, sometimes in considerable amounts, even though these gases are among the most potent greenhouse gases known; thus, for example, 1 kg of SF₆ has the same effects as 22.2 t of CO₂ [EC Regulation No. 842/2006].
- Gas tamponades are not allowed to be filled from the steel cylinder in the OR because steel cylinders have to be stored in a special steel cabinet for pressurized containers.
- The single doses available on the market have to be prepared in several steps before the surgeon can use them.

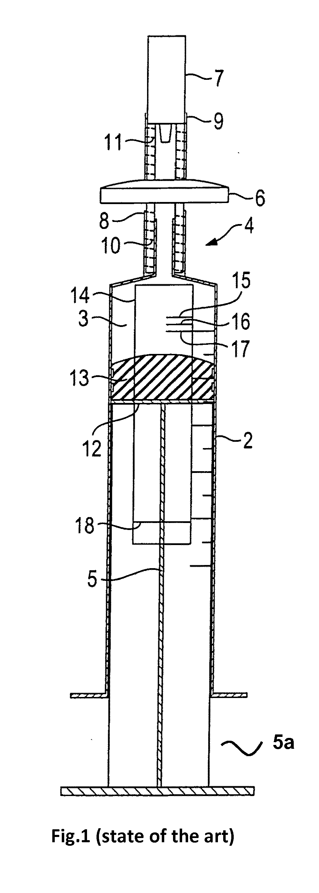

To solve these problems, European patent specification EP 2 319 566 B1, which represents the closest state of the art, discloses the following syringe for injecting a surgical gas, shown in Figure 1:
This is a syringe (1), in the form of a ready-to-use system,
   - with a syringe body (2) having
      - an inner chamber (3) and
      - a tip (4) connected to the inner chamber (3),
   - with a plunger rod (5) guided in a longitudinally displaceable manner in the inner chamber (3), and
   - with a sealing stopper (7),
   - characterized in that the syringe is in the form of a ready-to-use system,
   - the inner chamber (3) taking the form of a gas container and being filled with a heavy gas,
   - a syringe attachment filter (6) being fixed to the tip (4), and
   - the sealing stopper (7) being fixed to the syringe attachment filter (6), which seals the inner chamber (3) gas-tight.

Such a syringe is used as follows. First the inner chamber of the syringe body is filled with a heavy gas, such as SF₆, C₃F₈ or C₂F₆, in an amount greater than the desired (necessary) amount of heavy gas that is required to establish a desired mixing ratio of the heavy gas with air. The filled syringe is delivered in this form to the user (e.g. eye surgeon).

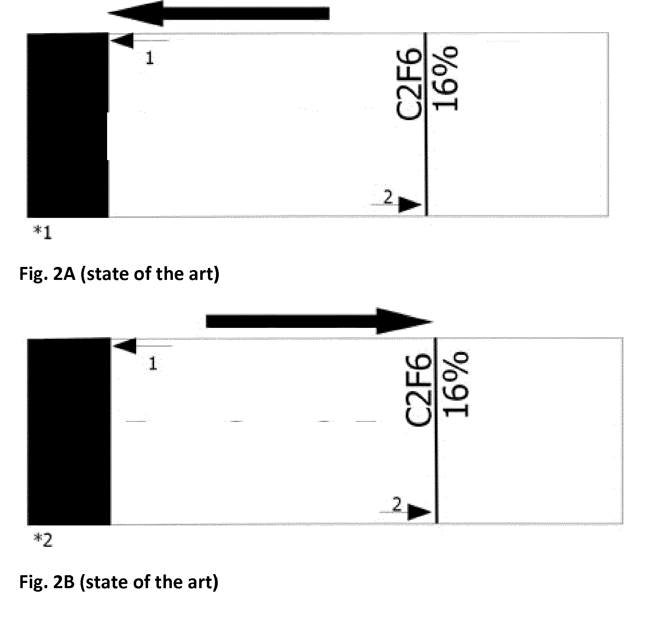

The necessary gas/air ratio must then still be established (by the user) before the syringe is used. This is done by pushing the syringe plunger forward (by means of the plunger rod 5) to a specified mark. Excess heavy gas is thereby expelled from the inner chamber (3). In the syringe of Figure 1 a label (14) with mixing ratio marks (15, 16 or 17) for different heavy gases, such as SF₆, C₃F₈ or C₂F₆, is affixed to the syringe body (2) for this purpose. In Figure 2A this mark is characterized by the reference (*1). The plunger is then pulled back to the second mark (reference (*2) in Figure 2B). This is how the desired mixing ratio is established (e.g. 16% C₂F₆/84% air, as shown in Figure 2). After the desired gas/air mixture has been established, the syringe attachment filter (6) is removed and a desired cannula, fitted with the protective cap, is attached. After the vitrectomy has been performed, the mixture of heavy gas and air is used as the medium for the long-term tamponade. The heavy gas has reached its full expansion about 72 hours after the vitrectomy has been performed and is not removed, but replaced with endogenous liquids and exhaled over a period of up to 30 days.

It would be desirable, however, to provide a ready-to-use syringe (i.e. a syringe in the form of a ready-to-use system) which allowed even easier handling for surgical use. In the case of prolonged storage of the syringe prior to use in the eye operation, it is also possible for air to diffuse into the inner chamber of the syringe body (plastic materials are known to be permeable to gases like oxygen or nitrogen), thereby altering the amount of heavy gas in the inner chamber. This could then lead to deviations from the desired mixing ratio of heavy gas to air.

The object of the present invention is therefore to provide a syringe of the type indicated in the pre-characterizing clause of Claim 1 which is capable of mitigating the disadvantages of the state of the art illustrated above and, in particular, is simple to operate.

### Detailed description of the invention

This object is achieved by the syringe packed inside a bag according to the invention which has the features of the independent claim 1. The independent method claim 11 is directed to a corresponding method of preparing a syringe filled with a predetermined amount of heavy gas in the form of a ready-to-use system which is packed inside a bag.

The respective subordinate claims contain advantageous additional forms of the invention.

In principle, various embodiments of the syringe according to the invention are covered by the present invention. Thus, preferably, the inner chamber of the syringe can be filled with a mixture of air and heavy gas in a predetermined mixing ratio, or alternatively just with heavy gas. Both embodiments are aspects of the same invention and solve the aforementioned technical problem insofar as they provide a syringe according to the invention which is ready to use and simple to operate and allows the preparation of a desired mixing ratio of heavy gas and air.

In one preferred embodiment, such a syringe is a syringe (1) in the form of a ready-to-use system, the syringe (1) being packed inside a bag (20) and comprising
- with a syringe body (2) having
   - an inner chamber (3) and
   - a tip (4) connected to the inner chamber (3),
- with a plunger (5a), guided in a longitudinally displaceable manner in the inner chamber (3), preferably with a plunger rod (5), and
- with a sealing stopper (7),
- a syringe attachment filter (6) being fixed to the tip (4), and
- the sealing stopper (7) being fixed to the syringe attachment filter (6), which seals the inner chamber (3) gas-tight, wherein the syringe body not carrying a mark (18) for the maximum plunger stroke and/or one or more mixing ratio marks (15, 16 or 17) for different heavy gases;
- characterized in that the inner chamber (3) is filled with a mixture of air and a heavy gas in a predetermined mixing ratio, the plunger rod (5) or plunger (5a), respectively, being positioned in a pulled back position such that the mixture of air and heavy gas present in the predetermined mixing ratio is capable of immediate administration; and
- wherein the bag (20) containing the syringe (1) is evacuated.

The expression "taking the form of a gas container" when used in this application means "designed as a gas container" and/or "used as a gas container", and implies that the inner chamber (3) preferably has characteristics which render it suitable for containing or holding back gas, e.g., that it can be closed in a gas-tight manner.
The expression "gas" in general comprises any substance in gaseous form, and in particular heavy gas and/or air as defined herein.

The sealing stopper is preferably fixed to the syringe attachment filter (6) and seals the inner chamber gas-tight, meaning that the sealing stopper (7) allows essentially no gas to exit and/or enter the inner chamber (3). The ready-to-use-system comprises a syringe attachment filter, and the sealing stopper is affixed to the syringe attachment filter, "sealing the inner chamber gas-tight". Gas may diffuse back and forth through the syringe attachment filter, but is prevented from entering or leaving the system by the sealing stopper.

Also disclosed herein are forms in which the syringe attachment filter 6 can also be omitted, the sealing stopper 7 being fixed to the syringe in such a way that it seals the inner chamber 3 gas-tight. This form is applicable to all the embodiments described herein, e.g., embodiments pertaining to the syringe or methods described herein.

"Seal gas-tight" means "seal in a manner such that essentially no gas is allowed to exit and/or enter". Those skilled in the art are aware of the fact that it is technically impossible to produce a completely gas-tight seal that totally prevents the exchange of individual gas molecules over an unlimited period of time. The expression therefore covers a small loss of gas from the inner chamber.

Other details, features and advantages of the invention are evident from the following detailed description of the invention and individual Examples and with the aid of the drawings, in which:
**Fig. 1** shows a ready-to-use syringe (syringe in the form of a ready-to-use system) known from European patent specification EP 2 319 566 B1.
**Fig. 2** illustrates, by way of a syringe filled with C₂F₆, the method of establishing the desired mixing ratio between the heavy gas and air in the syringe of Figure 1. **Fig. 2A** shows the first step, in which excess heavy gas is expelled from the inner chamber of the syringe body by pushing the syringe plunger up to the mark * 1, and the amount of heavy gas is reduced to the amount that is desired to establish the desired mixing ratio of heavy gas to air. **Fig. 2B** shows the second step, in which air is aspirated by moving the plunger back to the mark *2 in order to achieve the desired mixing ratio of heavy gas to air.

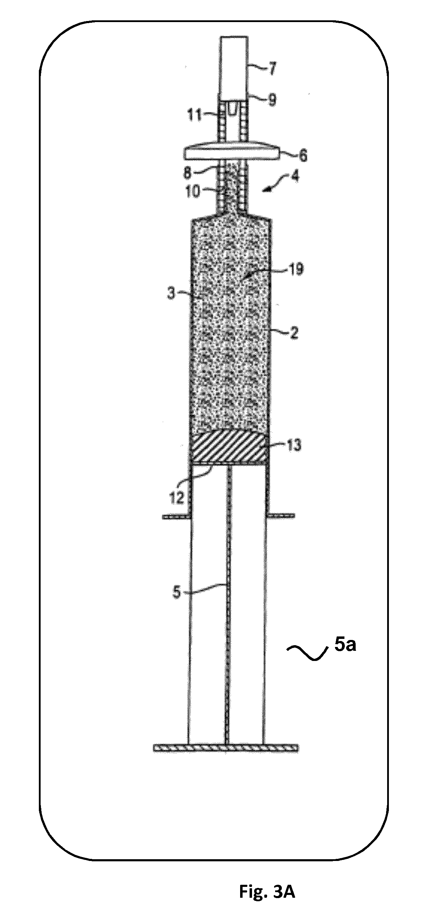

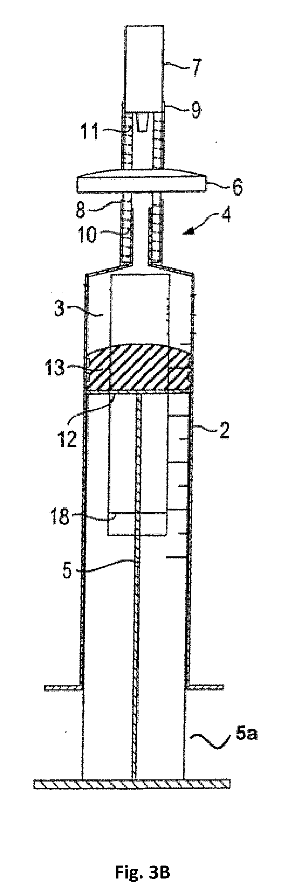

**Fig. 3** shows embodiments of a syringe packed inside a bag according to the invention. **Fig. 3A** illustrates a preferred embodiment of a syringe and of a bag according to the invention with the syringe housed inside, the inner chamber of the syringe containing a mixture of heavy gas and air. **Fig. 3B** shows an alternative embodiment of a syringe which does not form part of the invention.

One preferred embodiment of the present invention is based on filling the inner chamber 3 of the syringe, in the form of a gas container, at the outset with a heavy gas and air in a predetermined mixing ratio so that the syringe containing the predetermined mixing ratio of the mixture of air and heavy gas is capable of immediate use. In other words, the invention makes it possible, by establishing the desired mixing ratio of heavy gas to air, to omit both the steps shown in Fig. 2 that have to be performed by the user.

This preferred embodiment of the syringe according to the invention is substantially simpler to operate than syringes known from the state of the art, since the desired mixing ratio of heavy gas to air is established at the outset and no longer has to be established by the user. This in turn has the advantage that it is possible to omit a mark (18) for the maximum plunger stroke and/or mixing ratio marks (15, 16 or 17) for different heavy gases, such as SF₆, C₃F₈ or C₂F₆, on the syringe body.

In contrast to the invention, the inner chamber 3 of the syringe, in the form of a gas container, is filled at the outset with a predetermined amount of a heavy gas, the amount of heavy gas immediately giving a desired mixing ratio of the heavy gas with the aspirated air when the plunger rod 5 or plunger 5a, respectively, is directly pulled back to the mark 18 affixed to the syringe body for the maximum plunger stroke. This example makes it possible, by establishing the desired mixing ratio of heavy gas to air, to omit the first step shown in **Fig. 2**. This alternative to the syringe according to the invention accordingly makes it possible to establish the desired mixing ratio without the plunger rod 5 having been moved, prior to the aspiration of air, to a position in the inner chamber 3 where an excess amount of the heavy gas is expelled from the inner chamber 3 in order to establish the desired mixing ratio between the heavy gas and the air. Once again, this alternative embodiment is substantially simpler to operate than syringes known from the state of the art, since the desired mixing ratio of heavy gas to air can be established by pulling out the plunger 5a once and aspirating air.

In conformance with the above disclosure, all the embodiments of the syringe according to the invention accordingly have the advantage that it is possible to omit mixing ratio marks (15, 16 or 17) for different heavy gases, such as SF₆, C₃F₈ or C₂F₆, on the syringe body. The syringe according to the invention therefore also does without a label 14 to which mixing ratio marks (15, 16 or 17) for different heavy gases are affixed. This not only simplifies the design and production of the syringe, but also has the advantage that the user cannot inadvertently establish an incorrect mixing ratio by accidentally moving the plunger 5a, when expelling the excess heavy gas, to a mark intended for a gas other than the one being used. This potential source of error in use is thus excluded by the syringe according to the invention. The various embodiments accordingly have the advantage that they are simpler to operate than the syringes known from the state of the art and that an excess amount of heavy gas does not have to be used (i.e. actually wasted) in order to fill them.

An embodiment of the syringe according to the invention is illustrated in **Fig.3A**.

This shows embodiments of a syringe 1 according to the invention which have a cylindrical syringe body 2 comprising an inner chamber 3. The inner chamber 3 is connected to a tip 4, and a plunger rod 5 is guided in a longitudinally displaceable manner in the inner chamber 3.

As also illustrated in **Fig. 3A** and for explanatory purposes also in **Fig. 3B** , a syringe attachment filter 6 is fixed to the tip 4. The entire system is further sealed gas-tight with a sealing stopper 7, which in turn can be fixed to the outer end of the syringe attachment filter 6.

In one particularly preferred embodiment, the syringe attachment filter 6 has a first fixing section 8 for attachment to the tip 4 and a second fixing section 9 for securing the sealing stopper 7. Preferably, the syringe attachment filter 6 and the sealing stopper 7 can be fixed by means of screw connectors 10 and 11 respectively. In one particularly preferred embodiment, the screw connectors 10 and 11 each take the form of Luer lock connectors.

As also illustrated in **Fig. 3**, at its end region 12 located in the inner chamber 3, the plunger rod 5 of the plunger 5a has a rubber stopper 13, which is preferably siliconized.

In the syringe according to the invention shown in **Fig. 3A**, the plunger rod 5 or plunger 5a, respectively, is positioned in such a way that the mixture 19 of air and heavy gas present in the predetermined mixing ratio is capable of immediate administration. "Capable of immediate administration" means that the mixture of air and heavy gas is already present in the desired mixing ratio in the inner chamber 3 of the syringe body 2 and can be administered directly by the user, e.g. an ophthalmologist, for the therapeutic purpose of his choice without any further steps (such as the aspiration of air and expulsion of the excess amount of air to establish the desired mixing ratio of heavy gas to air). **Fig. 3A** also shows a bag 20 in which the syringe 1 has been housed (packed). The bag 20 is conventionally sealed air-tight and is also conventionally sterilized together with the syringe contained therein for the use of the syringe.

Apart from the conventional gaseous mixture of the earth's atmosphere, consisting mainly of the two gases nitrogen (around 78.08 vol%) and oxygen (around 20.95 vol%) and traces of argon, carbon dioxide, water vapour and other gases, the term "air" also includes synthetic mixtures of nitrogen and oxygen with other mixing ratios, e.g. 95 vol% of nitrogen and 5 vol% of oxygen, 80 vol% of nitrogen and 20 vol% of oxygen or 75 vol% of nitrogen and 25 vol% of oxygen. The term "air" also includes pure nitrogen in this context.

In contrast to the syringe shown in Fig. 1, no mark 18 is provided on the syringe body 2 for the maximum plunger stroke, nor are there any mixing ratio marks 15, 16 or 17 for different heavy gases, which, in the case of the syringe according to **Fig. 1**, can be affixed directly to the syringe body 2 or a label 14.

**Fig. 3B** shows an example of a syringe that does not form part of the invention in which a mark 18 is provided for the maximum plunger stroke. This mark 18 can be affixed either directly to the syringe body 2 or "indirectly" by way of a label (not shown in Figure 3). Here again there are no mixing ratio marks 15, 16 or 17 for different heavy gases, which, in the case of the syringe according to **Fig. 1**, can be affixed directly to the syringe body 2 or a label 14.

As already explained at the outset, the syringe 1 according to the invention can take the form of a disposable syringe.

In all the embodiments, the heavy gas used in the syringe can be any gas suitable for eye operations. In preferred embodiments, the heavy gas is SF₆, C₃F₈ or C₂F₆. In the latter case, in embodiments such as those shown in **Fig. 3A**, the predetermined amount of air and heavy gas in the inner chamber 3 is preferably proportioned to give the following mixing ratio:
- for SF₆: 20% gas / 80% air,
- for C₂F₆: 16% gas / 84% air and
- for C₃F₈: 12% gas / 88% air.

Not according to the invention, as shown in **Fig. 3B**, the predetermined amount of heavy gas in the inner chamber 3 is preferably proportioned to give the aforementioned mixing ratios of the heavy gas with the aspirated air after the plunger rod has been pulled back.

For all the embodiments with a (fillable) inner chamber volume of 100 ml, the predetermined amount of e.g. SF₆ is 20 ml, the amount of C₂F₆ is 16 ml and the amount of C₃F₈ is 12 ml. For smaller syringe volumes, e.g. 50 ml filling volume of the inner chamber 3, the amounts used are to be adapted proportionately. However, it is also possible to introduce smaller amounts of heavy gas. It is possible, for example, to use only 16 ml of SF₆ (based on a syringe with a filling volume of the inner chamber 3 of 100 ml) and 84 ml of air, i.e. a mixing ratio of 16% heavy gas/84% air for subsequent surgical use, e.g. in a vitrectomy. The present example includes preferred embodiments in which 16 ml of SF₆ and 84 ml of air are present as the gaseous mixture in a syringe according to the invention and can be administered immediately. In the example in which only 16 ml of SF₆ are present in the syringe 84 ml of air are aspirated by directly pulling the plunger rod back. In this context, attention is drawn once again to the difference from the syringe known from European patent specification EP 2 319 566 B1. Thus, when using SF₆ as the heavy gas, the syringe known from EP 2 319 566 B1 with a filling volume of the inner chamber 3 of 100 ml is filled with 30 ml of SF₆ and delivered filled with this amount, so, after the excess heavy gas has been expelled and air aspirated (as shown in **Fig. 2**), 20 ml of SF₆ remain in the inner chamber 3 of the syringe and a mixing ratio of 20% gas/80% air is established. By contrast, the syringe according to the invention is filled at the outset with the desired 20 ml of SF₆ and 80 ml of air. In general terms, in the present invention, the syringe is filled at the outset with the amount of heavy gas and air, which is required for the desired mixing ratio of heavy gas with air. This amount is also referred to here as the "predetermined amount" of heavy gas. "Ready-to-use" implies that the syringe according to the invention is a ready-to-use system that does not require additional assembling and/or filling from an external gas container. The syringe according to the invention can thus be used directly in the manner described herein. This comprises, e.g. when using the syringe according to the invention in medical procedures, the attachment of a cannula or a tube immediately prior to injection of the heavy gas/air mixture.

The syringe according to the invention is packed in a bag that is preferably gas-tight.

In conformance with the above disclosure, methods of filling a syringe with a predetermined amount of a heavy gas or a predetermined amount of a heavy gas and air (heavy gas/air mixture in a predetermined mixing ratio) are also disclosed here. The syringe in this method is a syringe in the form of a ready-to-use system with
- a syringe body 2 having
   - an inner chamber 3 and
   - a tip 4 connected to the inner chamber 3,
- a plunger (5a), guided in a longitudinally displaceable manner in the inner chamber (3), preferably with a plunger rod (5), and
- a sealing stopper 7,
- the inner chamber 3 taking the form of a gas container and being fillable with air and a heavy gas in a predetermined gas mixing ratio,
- a syringe attachment filter 6 being fixed to the tip 4, and
- the sealing stopper 7 being fixed to the syringe attachment filter 6, which seals the inner chamber 3 gas-tight,
- the method comprising:
- positioning of the plunger rod 5 or plunger 5a, respectively, in such a way that the inner chamber 3 can be filled with a predetermined mixing ratio of a mixture of air and a heavy gas, and
- filling of the inner chamber with amounts of air and heavy gas which are proportioned to give the desired mixing ratio so that the mixture of air and heavy gas is capable of immediate administration.

In conformance with the above disclosure, the heavy gas used can be SF₆, C₃F₈ or C₂F₆. The predetermined amount of heavy gas in the inner chamber 3 can be proportioned in such a way as to give the following mixing ratio of the heavy gas to air
- for SF₆: 20% gas/80% air,
- for C₂F₆: 16% gas/84% air and
- for C₃F₈: 12% gas/88% air.

In conformance with the above disclosure, in contrast to the known syringe from Fig. 1, the syringe body 2 of the syringe 1 according to the invention preferably has no mark 18 for the maximum plunger stroke and/or no mixing ratio marks 15, 16 or 17 for different heavy gases.

The method according to the invention comprises also the step the step of introducing the filled syringe into a bag and sealing the bag (gas-tight). In some embodiments, the bag used for this purpose is a bag made of a plastic material that is acceptable for pharmaceutical packs. Such a bag can be a sterile bag. The resulting system made up of a bag and a syringe packed inside is then conventionally sterilized, e.g. by autoclaving, with UV, X rays or gamma radiation or with formaldehyde, ethylene oxide, ozone or hydrogen peroxide, and shipped in sterile form to the user.

The bag with the syringe inside is also evacuated by withdrawing air from it. For this purpose the bag is conventionally designed so as to be suitable for evacuation. This can imply, inter alia, that the bag is made of a vacuum-tight material, e.g. polyamide, polyethylene or laminated film.

Alternatively, it is also possible to use a bag that is not suitable for evacuation. In this case the bag can first be sterilized together with the syringe inside, as described above. The sterilized bag together with the syringe inside can then be packed in a second bag that is suitable for evacuation ("vacuum bag"). The vacuum bag can then be evacuated.

Not according to the present invention an alternative method in which the syringe is a syringe in the form of a ready-to-use system would be possible - with a syringe body 2 having
- an inner chamber 3 and
- a tip 4 connected to the inner chamber 3,
   - with a plunger (5a), guided in a longitudinally displaceable manner in the inner chamber (3), preferably with a plunger rod (5),
   - with a sealing stopper 7, and
   - with a mark 18 affixed to the syringe body 2 for the maximum plunger stroke,
   - the inner chamber 3 taking the form of a gas container and being fillable with a predetermined amount of a heavy gas,
   - a syringe attachment filter 6 being fixed to the tip 4, and
   - the sealing stopper 7 being fixed to the syringe attachment filter 6, which seals the inner chamber 3 gas-tight.

The method comprises the step of aspirating air into the inner chamber 3, containing a predetermined amount of a heavy gas, by directly pulling the plunger rod 5 or plunger 5a, respectively, back to the mark 18 for the maximum plunger stroke to give a desired mixing ratio of the heavy gas with the aspirated air without the plunger rod 5 or plunger 5a, respectively, having been moved, prior to the aspiration of air, to a position in the inner chamber 3 where an excess amount of the heavy gas is expelled from the inner chamber 3 in order to establish the desired mixing ratio between the heavy gas and the air.

The method disclosed here further comprises the step, prior to the aspiration of air, of filling the inner chamber 3 of the syringe body with a predetermined amount of the heavy gas.

In conformance with the above disclosure, the heavy gas used can be SF₆, C₃F₈ or C₂F₆. In this embodiment, the predetermined amount of heavy gas in the inner chamber 3 can be proportioned in such a way as to give the following mixing ratio of the heavy gas to the aspirated air after aspiration of the air by pulling the plunger rod back:
- for SF₆: 20% gas/80% air,
- for C₂F₆: 16% gas/84% air and
- for C₃F₈: 12% gas/88% air.

In all the methods described here, in preferred embodiments with a volume of the inner chamber 3 of 100 ml, the predetermined (introduced) amount of SF₆ is 20 ml, the amount of C₂F₆ is 16 ml and the amount of C₃F₈ is 12 ml.

All the methods described here are methods of preparing a mixture of a heavy gas and air in a desired mixing ratio and at the same time methods of administering this mixture for medical purposes, e.g. an eye operation.

Those skilled in the art are aware that the syringe according to the invention is preferably sterile, especially for medical purposes. Provision is therefore made for the syringe according to the invention to be able to be prepared and filled under sterile conditions, and/or to be sterilized after preparation and filling, as described above.

Using the syringe according to the invention of one of the embodiments described above, a heavy gas/air mixture is administered to a patient's vitreous humour and/or vitreous body in order to effect a so-called gas tamponade (filling of the vitreous chamber). The gas tamponade is intended to press the retina on to the substrate. The retina is thus stabilized by the gas tamponade and smoothed out as required, and/or the closure of a macular foramen is supported.

The gas tamponade disappears from the eye over a period of approx. 1 week (air) to several weeks (heavy gas). The vitreous chamber is then refilled with collyrium, which is regularly produced by the ciliary body and replaces the vitreous humour.
The present invention therefore describes the use of a heavy gas for the preparation of a product, preferably a medical product such as, in particular, the syringe according to the invention. According to the invention, a predetermined amount of a heavy gas and air is filled into the product, as described herein, so that it can then be administered immediately by the user (e.g. eye surgeon) in this predetermined mixing ratio of heavy gas to air.

The present invention further describes the use of a heavy gas for a device, e.g. a medical product, especially a syringe for injecting a surgical heavy gas into the vitreous humour and/or the vitreous body of an eye in order to prevent and/or treat retinal detachments, e.g. retinal detachments with giant tears, retinal detachments with proliferation, retinal detachments in cases of proliferative diabetic retinopathy (PDR), and traumatic retinal detachments. In other words, when used, the device is preferably filled with the heavy gas and air, as described herein, so that retinal detachments are prevented and/or treated with the ready-to-use system described herein.

In addition to the above written disclosure of the invention, explicit reference is hereby made to the drawings thereof and the explanations relating to the state of the art in Figures 1 to 3.

### List of reference numbers

- 1: syringe
- 2: syringe body
- 3: inner chamber
- 4: tip
- 5: plunger rod
- 5a: plunger
- 6: syringe attachment filter
- 7: sealing stopper
- 8: fixing section
- 9: fixing section
- 10, 11: screw connectors
- 13: rubber stopper
- 14: label
- 15, 16, 17: mixing ratio marks
- 18: mark for maximum plunger stroke
- 19: mixture of air and heavy gas
- 20: bag

## Claims

1. Syringe (1) in the form of a ready-to-use system, the syringe (1) being packed inside a bag (20) and comprising a syringe body (2) having
• an inner chamber (3) taking the form of a gas container and
• a tip (4) connected to the inner chamber (3),
- with a plunger (5a), guided in a longitudinally displaceable manner in the inner chamber (3), preferably with a plunger rod (5), and
- with a sealing stopper (7),
- a syringe attachment filter (6) being fixed to the tip (4), and
- the sealing stopper (7) being fixed to the syringe attachment filter (6), which seals the inner chamber (3) gas-tight, and
- wherein the syringe body (12) does not carry a mark (18) for the maximum plunger stroke and/or one or more mixing ratio marks (15, 16 or 17) for different heavy gases;
- **characterized in that** the inner chamber (3) is filled with a mixture of air and a heavy gas in a predetermined mixing ratio, the plunger rod (5) or plunger (5a), respectively, being positioned in such a way that the mixture of air and heavy gas present in the predetermined mixing ratio is capable of immediate administration; and
- wherein the bag (20) containing the syringe (1) is evacuated.

2. Syringe according to Claim 1 wherein the syringe attachment filter (6) has a first fixing section (8) for attachment to the tip (4) and a second fixing section (9) for securing the sealing stopper (7).

3. Syringe according to Claim 1 or 2 wherein the syringe attachment filter (6) and the sealing stopper (7) are fixed by means of screw connectors (10 and 11 respectively).

4. Syringe according to Claim 3 wherein the screw connectors (10 and 11) take the form of Luer lock connectors.

5. Syringe according to Claims 1 to 4 wherein, at its end region (12) located in the inner chamber (3), the plunger rod (5) is provided with a rubber stopper (13).

6. Syringe according to Claim 5 wherein the rubber stopper (13) is siliconized.

7. Syringe according to any one of Claims 1 to 6 which takes the form of a disposable syringe.

8. Syringe according to any one of Claims 1 to 7 wherein the heavy gas is SF₆, C₃F₈ or C₂F₆.

9. Syringe according to Claim 8 wherein the heavy gas and air are present in the inner chamber (3) in the following mixing ratio:
- for SF₆: 20% gas/80% air,
- for C₂F₆: 16% gas/84% air and
- for C₃F₈: 12% gas/88% air.

10. Syringe according to Claim 9 wherein, with a volume of the inner chamber (3) of 100 ml, the amount of SF₆ is 20 ml, the amount of C₂F₆ is 16 ml and the amount of C₃F₈ is 12 ml, the remainder being air.

11. Method of preparing a syringe (1) filled with a predetermined amount of a heavy gas in the form of a ready-to-use system which is packed inside a bag (20), the syringe (1) comprising a syringe body (2) having
• an inner chamber (3) and
• a tip (4) connected to the inner chamber (3),
- a plunger (5a), guided in a longitudinally displaceable manner in the inner chamber (3), preferably with a plunger rod (5), and
- a sealing stopper (7),
- the inner chamber (3) taking the form of a gas container and being fillable with air and a heavy gas in a predetermined gas mixing ratio,
- a syringe attachment filter (6) being fixed to the tip (4), and
- the sealing stopper (7) being fixed to the syringe attachment filter (6), which seals the inner chamber (3) gas-tight,
comprising the following steps:
- positioning of the plunger rod (5) or plunger (5a), respectively, in such a way that the inner chamber (3) can be filled with a predetermined mixing ratio of a mixture of air and a heavy gas, and
- filling of the inner chamber with amounts of air and heavy gas which are proportioned to give the desired mixing ratio so that the mixture of air and heavy gas is capable of immediate administration;
- placing the syringe (1) inside the bag (20);
- evacuating the bag (20) by withdrawing air from it.

12. Syringe according to any one of Claims 1-10 and 13 for the treatment and/or prevention of retinal detachments.

## Patentansprüche

1. Spritze (1) in Form eines gebrauchsfertigen Systems, wobei die Spritze (1) in einem Beutel (20) verpackt ist und einen Spritzenkörper (2) aufweist, der folgendes aufweist
• eine innere Kammer (3), die die Form eines Gasbehälters annimmt, und
• eine Spitze (4), die mit der inneren Kammer (3) verbunden ist,
- mit einem Kolben (5a), der in einer längsverschiebbaren Weise in der inneren Kammer (3) geführt ist, vorzugsweise mit einer Kolbenstange (5), und
- mit einem Dichtstopfen (7),
- einem Spritzen-Vorsatzfilter (6), der an der Spitze (4) befestigt ist, und
- wobei der Dichtstopfen (7) an dem Spritzen-Vorsatzfilter (6) befestigt ist, der die innere Kammer (3) gasdicht verschließt, und
- wobei der Spritzenkörper (12) keine Markierung (18) für den maximalen Kolbenhub und/oder eine oder mehrere Markierungen (15, 16 oder 17) für das Mischverhältnis für verschiedene schwere Gase trägt;
- **dadurch gekennzeichnet, dass** die innere Kammer (3) mit einer Mischung aus Luft und einem schweren Gas in einem vorbestimmten Mischverhältnis gefüllt ist, wobei die Kolbenstange (5) bzw. der Kolben (5a) so positioniert sind, dass die in dem vorbestimmten Mischverhältnis vorhandene Mischung aus Luft und schwerem Gas sofort verabreicht werden kann; und
- wobei der die Spritze (1) enthaltende Beutel (20) evakuiert ist.

2. Spritze nach Anspruch 1, wobei der Spritzen-Vorsatzfilter (6) einen ersten Befestigungsabschnitt (8) zur Befestigung an der Spitze (4) und einen zweiten Befestigungsabschnitt (9) zum Befestigen des Dichtstopfens (7) aufweist.

3. Spritze nach Anspruch 1 oder 2, wobei der Spritzen-Vorsatzfilter (6) und der Dichtstopfen (7) mittels Schraubverbindern (10 bzw. 11) befestigt sind.

4. Spritze nach Anspruch 3, wobei die Schraubverbinder (10 und 11) die Form von Luer-Lock-Verbindern annehmen.

5. Spritze nach Anspruch 1 bis 4, wobei die Kolbenstange (5) an ihrem in der inneren Kammer (3) angeordneten Endbereich (12) mit einem Gummistopfen (13) versehen ist.

6. Spritze nach Anspruch 5, wobei der Gummistopfen (13) silikonisiert ist.

7. Spritze nach einem der Ansprüche 1 bis 6, die die Form einer Einmalspritze annimmt.

8. Spritze nach einem der Ansprüche 1 bis 7, wobei das schwere Gas SF₆, C₃F₈ oder C₂F₆ ist.

9. Spritze nach Anspruch 8, wobei das schwere Gas und die Luft in der inneren Kammer (3) in dem folgenden Mischverhältnis vorhanden sind:
- für SF₆: 20% Gas/80% Luft,
- für C₂F₆: 16% Gas/84% Luft und
- für C₃F₈: 12% Gas/88% Luft.

10. Spritze nach Anspruch 9, wobei, bei einem Volumen der inneren Kammer (3) von 100 ml, die Menge an SF₆ 20ml, die Menge an C₂F₆ 16 ml und die Menge an C₃F₈ 12 ml beträgt, wobei der Rest Luft ist.

11. Verfahren zum Vorbereiten einer Spritze (1), gefüllt mit einer vorbestimmten Menge eines schweren Gases in Form eines gebrauchsfertigen Systems, das in einem Beutel (20) verpackt ist, wobei die Spritze (1) einen Spritzenkörper (2) aufweist, der folgendes aufweist
• eine innere Kammer (3) und
• eine Spitze (4), die mit der inneren Kammer (3) verbunden ist,
- einen Kolben (5a), der in einer längsverschiebbaren Weise in der inneren Kammer (3) geführt ist, vorzugsweise mit einer Kolben-stange (5), und
- einen Dichtstopfen (7),
- wobei die innere Kammer (3) die Form eines Gasbehälters annimmt und mit Luft und einem schweren Gas in einem vorbestimmten Gasmischungsverhältnis befüllbar ist,
- einen Spritzen-Vorsatzfilter (6), der an der Spitze (4) befestigt ist, und wobei
- der Dichtstopfen (7) an dem Spritzen-Vorsatzfilter (6) befestigt ist, der die innere Kammer (3) gasdicht abdichtet,
umfassend die folgenden Schritte:
- Positionieren der Kolbenstange (5) bzw. des Kolbens (5a) so, dass die innere Kammer (3) mit einem vorbestimmten Mischverhältnis einer Mischung aus Luft und einem schweren Gas befüllt werden kann, und
- Füllen der inneren Kammer mit Mengen an Luft und schwerem Gas, die so dosiert sind, dass sie das gewünschte Mischverhältnis ergeben, so dass die Mischung aus Luft und schwerem Gas sofort verabreicht werden kann;
- Einbringen der Spritze (1) in den Beutel (20);
- Evakuieren des Beutels(20), indem ihm Luft entzogen wird.

12. Spritze nach einem der Ansprüche 1-10 und 13 zur Behandlung und/oder Vorbeugung von Netzhautablösungen.

## Revendications

1. Seringue (1) sous la forme d'un système prêt à l'emploi, la seringue (1) étant emballée dans un sac (20) et comportant un corps de seringue (2) possédant
• une chambre interne (3) prenant la forme d'un récipient de gaz et
• une pointe (4) liée à la chambre interne (3),
- avec un piston (5a) guidé de manière déplaçable longitudinalement dans la chambre interne (3), de préférence avec une tige de piston (5), et
- avec un bouchon d'étanchéité (7),
- un filtre additionnel de seringue (6) étant fixé à la pointe (4), et
- le bouchon d'étanchéité (7) étant fixé au filtre additionnel de seringue (6) qui ferme et rend étanche au gaz la chambre interne (3), et
- le corps de seringue (12) ne comportant pas un repère (18) pour la course maximum du piston et/ou un ou plusieurs repères (15, 16 ou 17) pour le rapport de mélange pour des gaz lourds différents;
- **caractérisé en ce que** la chambre interne (3) est remplie d'un mélange d'air et d'un gaz lourd dans un rapport de mélange prédéfini, la tige de piston (5) et/ou le piston (5a) étant positionné de manière que le mélange d'air et du gaz lourd présent dans le rapport de mélange prédéfini peut être administré immédiatement; et
- le sac (20) comportant la seringue (1) étant évacué.

2. Seringue selon la revendication 1, le filtre additionnel de seringue (6) ayant une première section de fixation (8) pour la fixation à la pointe (4) et une deuxième section de fixation (9) pour fixer le bouchon d'étanchéité (7).

3. Seringue selon la revendication 1 ou 2, le filtre additionnel de seringue (6) et le bouchon d'étanchéité (7) étant fixé au moyen des connecteurs à vis (10 et/ou 11).

4. Seringue selon la revendication 3, les connecteurs à vis (10 et 11) prenant la forme des connecteurs Luer Lock.

5. Seringue selon la revendication 1 à 4, la tige de piston (5) étant pourvue d'un bouchon en caoutchouc (13) à sa zone d'extrémité (12) située dans la chambre interne (3).

6. Seringue selon la revendication 5, le bouchon en caoutchouc (13) étant siliconé.

7. Seringue selon l'une quelconque des revendications 1 à 6 qui prend la forme d'une seringue jetable.

8. Seringue selon l'une quelconque des revendications 1 à 7, le gaz lourd étant SF₆, C₃F₈ ou C₂F₆.

9. Seringue selon la revendication 8, le gaz lourd et de l'air étant présent dans la chambre interne (3) dans le rapport de mélange suivant:
- pour SF₆: 20% du gaz/80% de l'air,
- pour C₂F₆: 16% du gaz/84% de l'air et
- pour C₃F₈: 12% du gaz/88% de l'air.

10. Seringue selon la revendication 9, dans un volume de la chambre interne (3) de 100 ml, le montant de SF₆ étant de 20ml, le montant de C₂F₆ étant de 16 ml et le montant de C₃F₈ étant de 12 ml, le reste étant de l'air.

11. Procédé de préparation d'une seringue (1) remplie avec un montant prédéfini d'un gaz lourd sous la forme d'un système prêt à l'emploi qui est emballée dans un sac (20), la seringue comportant un corps de seringue (2) possédant
• une chambre interne (3) et
• une pointe (4) liée à la chambre interne (3),
- un piston (5a) guidé de manière déplaçable longitudinalement dans la chambre interne (3), de préférence avec une tige de piston (5), et
- un bouchon d'étanchéité (7),
- la chambre interne (3) prenant la forme d'un récipient de gaz et pouvant être remplie avec de l'air et d'un gaz lourd dans un rapport de mélange prédéfini,
- un filtre additionnel de seringue (6) étant fixé à la pointe (4), et
- le bouchon d'étanchéité (7) étant fixé au filtre additionnel de seringue (6) qui ferme et rend étanche au gaz la chambre interne (3),
comportant les étapes suivantes:
- positionnement de la tige de piston (5) et/ou du piston (5a) de manière que la chambre interne (3) peut être remplie avec un rapport de mélange prédéfini d'un mélange de l'air et d'un gaz lourd, et
- remplissage de la chambre interne (3) avec des montants de l'air et du gaz lourd proportionnés à donner le rapport de mélange demandé de manière que le mélange de l'air et du gaz lourd peut être administré immédiatement;
- placement de la seringue (1) dans le sac (20);
- évacuation du sac (20) en retirant de l'air.

12. Seringue selon l'une quelconque des revendications 1-10 et 13 pour le traitement et/ou la prévention des décollements de rétine.
